# EUROPEAN PATENT APPLICATION

(11) **EP 3 488 798 A1**
(43) Date of publication of application: **29.05.2019**
(21) Application number: 18208630.6
(22) Date of filing: 27.11.2018
(51) Int. Cl.: A61B 17/122

(54) **SURGICAL LIGATION CLIP WITH TISSUE STOP MEMBER**

(30) Priority: 28.11.2017 US 201762591721 P; 24.09.2018 US 201816139581
(71) Applicant: Covidien LP, Mansfield, MA 02048 (US)
(72) Inventor: KAMARAJ, Raja, 500055 Hyderabad (IN); MOHANASUNDARAM, Suresh Kumar Prema, 602024 Chennai (IN); GEORGE, Sabastian Koduthully, 50050 Hyderabad (IN)
(74) Representative: Maschio, Antonio

(57) **Abstract**

A surgical ligation clip includes first and second jaws that are connected at their proximal ends to each other by a living hinge. Each of the jaws includes a clamping surface that is in opposition to the clamping surface of the other jaw. In order to prevent the body vessel from moving proximally beyond the clamping surfaces of the first and second jaws, a tissue stop is formed at a proximal end of the clamping surfaces of the surgical ligation clip. In embodiments, the tissue stop includes a first stop member that extends from the proximal end of the clamping surface of the first jaw towards the second jaw and a second stop member that extends from the proximal end of the clamping surface of the second jaw member towards the first jaw.

## Description

### CROSS-REFERENCE TO RELATED APPLICATION

This application claims the benefit of and priority to U.S. Provisional Patent Application No. 62/591,721 filed November 28, 2017, the entire disclosure of which is incorporated by reference herein.

### BACKGROUND

### 1. Technical Description

The present disclosure is directed to surgical ligation clips and, more particularly, to surgical ligation clips with a proximally located tissue stop feature.

### 2. Background of Related Art

Surgical ligation clips are commonly used during laparoscopic procedures including, e.g., appendectomies, cholecystectomies, nephrectomies and splenectomies to seal a body vessel. These surgical ligation clips are typically formed from a bio-compatible, non-absorbable, non-conductive, polymer and include a pair of jaws that have clamping surfaces and are joined to each other at their proximal ends by a living hinge. The jaws are movable in relation to each other about the living hinge from an unclamped position to a clamped position. In the clamped position, the clamping surfaces of the jaws are positioned to compress tissue positioned between the jaws to seal a lumen defined by the tissue.

In known surgical ligation clips, the living hinge defines an area at the proximal end of the jaws that has a reduced thickness that is not configured to fully compress tissue when the jaws are in the clamped position. As such, when tissue is positioned in the area of the living hinge proximally of the clamping surfaces of the jaws, the tissue may not be fully compressed. Thus, fluid may continue to flow through a lumen defined by the tissue when the surgical ligation clip is clamped about the tissue.

A continuing need exists in the surgical arts for a surgical ligation clip that has improved ligation characteristics.

### SUMMARY

One aspect of the disclosure is directed to a surgical ligation clip including a first jaw having a distal end, a proximal end, and a first clamping surface and a second jaw having a distal end, a proximal end, and a second clamping surface. The second clamping surface of the second jaw is in opposition to the first clamping surface of the first jaw. A living hinge is provided to pivotably couple the proximal end of the first jaw to the proximal end of the second jaw. The first and second jaws are movable in relation to each other between an unclamped position and a clamped position. A tissue stop is supported on the surgical ligation clip and is positioned to prevent tissue from moving proximally between the first and second jaws beyond the first and second clamping surfaces when the first and second jaws are in the unclamped position.

In some embodiments, the tissue stop includes a first stop member supported on the first jaw that extends from a proximal end of the first clamping surface towards the second jaw.

In certain embodiments, the tissue stop includes a second stop member that extends from a proximal end of the second clamping surface of the second jaw towards the first jaw.

In embodiments, the first stop member is positioned to slidably engage the second stop member when the first and second jaws are moved in relation to each other from the unclamped position to the clamped position.

In some embodiments, the first jaw includes a first locking element and the second jaw member includes a second locking element, wherein the first locking element is movable into engagement with the second locking element to retain the first and second jaws in the clamped position.

In certain embodiments, the distal end of the first jaw includes a head that supports the first locking element.

In embodiments, the head includes a pointed leading end that is configured to penetrate tissue.

In some embodiments, each of the first and second jaws includes a pair of bosses and each of the pair of bosses is configured to engage an applicator to apply the surgical ligation clip to tissue.

In certain embodiments, each of the pair of bosses projects outwardly beyond an outer surface of one of the first and second jaws.

In embodiments, the clamping surfaces of the first and second jaws include a non-slip structure.

In some embodiments, the non-slip structure of the first clamping surface includes triangular protrusions that are spaced along opposite sides of the clamping surface and are axially offset from adjacent protrusions.

In certain embodiments, the triangular protrusions define a centrally located V-shaped recess that extends along at least a portion of the first clamping surface of the first jaw.

In embodiments, the second jaw includes a centrally located triangular protrusion that is positioned along at least a portion of the length of the second jaw, wherein the central triangular protrusion is positioned to be received in the centrally located V-shaped recess defined by the protrusions of the first jaw.

In some embodiments, the surgical ligation clip is formed from a biocompatible polymeric material.

In certain embodiments, the biocompatible polymeric material is selected from the group consisting of acetal polyoxymethylene (POM), polyethylene terephthalate (PET), polybutylene terephthalate (PBT), polyoxymethylene, polyetheretherketone (PEEK), polypropylene, and polyethylene.

In embodiments, the surgical ligation clip is formed from a biocompatible material selected from the group consisting of metals, plastics and composites.

### BRIEF DESCRIPTION OF THE DRAWINGS

Various embodiments of the presently disclosed surgical ligation clip are described herein below with reference to the drawings, wherein:
FIG. 1 is a side perspective view of an exemplary embodiment of the presently disclosed surgical ligation clip in an unclamped position;
FIG. 2 is an enlarged view of the indicated area of detail shown in FIG. 1;
FIG. 3 is a side perspective view of the presently disclosed surgical ligation clip shown in FIG. 1 in a clamped position;
FIG. 4 is an enlarged view of the indicated area of detail shown in FIG. 3;
FIG. 5 is a side view of the surgical ligation clip shown in FIG. 1 in the unclamped position and a body vessel positioned between first and second jaws of the surgical ligation clip;
FIG. 6 is a side view of the surgical ligation clip shown in FIG. 1 in the clamped position about the body; and
FIG. 7 is an enlarged view of the indicated area of detail shown in FIG. 3.

### DETAILED DESCRIPTION OF EMBODIMENTS

The presently disclosed surgical ligation clip will now be described in detail with reference to the drawings in which like reference numerals designate identical or corresponding elements in each of the several views. However, it is to be understood that the disclosed embodiments are merely exemplary of the disclosure and may be embodied in various forms. Well-known functions or constructions are not described in detail to avoid obscuring the present disclosure in unnecessary detail. Therefore, specific structural and functional details disclosed herein are not to be interpreted as limiting, but merely as a basis for the claims and as a representative basis for teaching one skilled in the art to variously employ the present disclosure in virtually any appropriately detailed structure.

In this description, the term "proximal" is used generally to refer to that portion of the device that is closer to a clinician, while the term "distal" is used generally to refer to that portion of the device that is farther from the clinician. In addition, the term "clinician" is used generally to refer to medical personnel including doctors, nurses, and support personnel.

The presently disclosed surgical ligation clip includes first and second jaws that are connected at their proximal ends to each other by a living hinge. Each of the jaws includes a clamping surface that is in opposition to the clamping surface of the other jaw. The first and second jaws are movable in relation to each other between unclamped and clamped positions to seal a lumen of a body vessel positioned between the first and second jaws. In order to prevent the body vessel from moving proximally beyond the clamping surfaces of the first and second jaws, a tissue stop is formed at a proximal end of the clamping surfaces of the surgical ligation clip. In embodiments, the tissue stop includes a first stop member that extends from the proximal end of the clamping surface of the first jaw towards the second jaw and a second stop member that extends from the proximal end of the clamping surface of the second jaw member towards the first jaw.

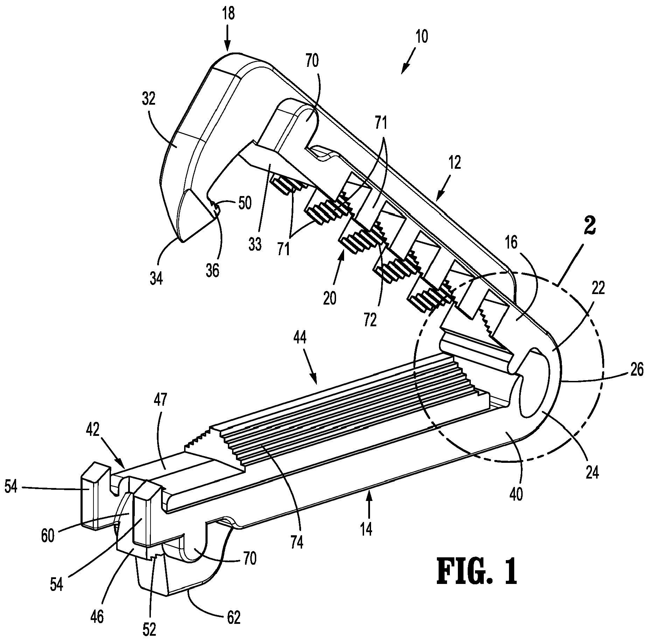

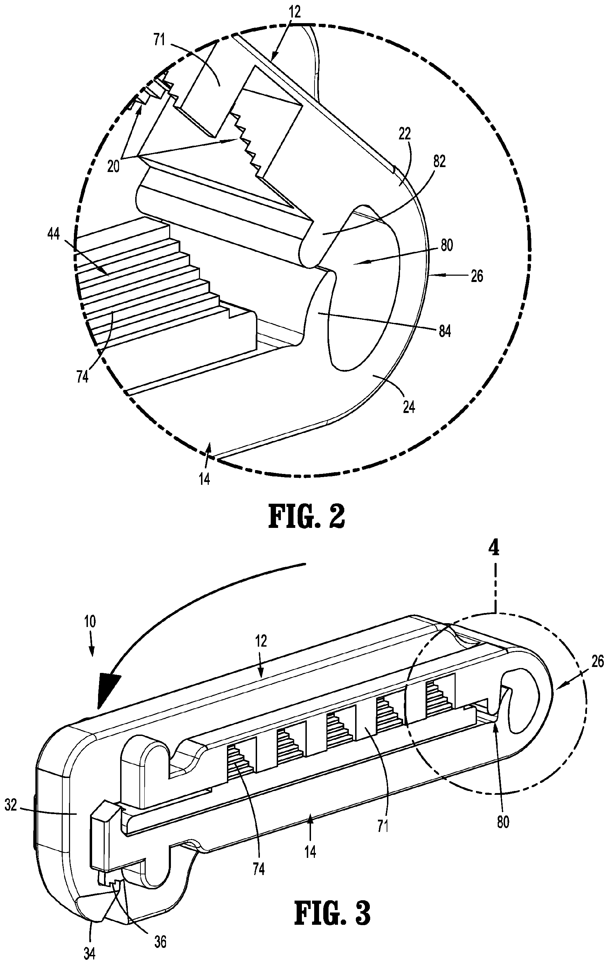

Referring to FIG. 1, an exemplary embodiment of the presently disclosed surgical ligation clip is shown generally as 10 and includes a first jaw 12 and a second jaw 14. The first jaw 12 includes a proximal end 16, a distal end 18, and a clamping surface 20. The proximal end 16 of the first jaw 12 includes an area of reduced thickness 22 that is connected to an area of reduced thickness 24 of the second jaw 14 to define a living hinge 26. The living hinge 26 connects the first jaw 12 to the second jaw 12 to facilitate movement of the first and second jaws 12, 14 in relation to each other between an unclamped position (FIG. 1) and a clamped position (FIG. 3).

The distal end 18 of the first jaw 12 includes a head 32 and a flat 33 positioned distally of the clamping surface 20. In embodiments, the head 32 includes a sharpened and/or pointed leading edge 34 that is configured to penetrate tissue as the tissue is clamped between the first and second jaws 12, 14. The head 32 also includes a first locking element 36 that is described in further detail below.

The second jaw 14 includes a proximal end 40, a distal end 42, and a clamping surface 44. As discussed above, the proximal end 40 of the second jaw 14 includes the area of reduced thickness 24 that forms the living hinge 26. The distal end 42 of the second jaw 14 includes a second locking element 46. In embodiments, the second locking element 46 is configured to releasably engage the first locking element 36 of the first jaw 12 to secure the first and second jaws 12, 14 in the clamped position (FIG. 3). In embodiments, the first locking element 36 of the first jaw 12 includes teeth 50 that are positioned and configured to engage teeth 52 on the second locking element 46 of the second jaw 14 to secure the first and second jaws 12, 14 in the clamped position.

The distal end 42 of the second jaw 14 supports the second locking element 46, and includes a flat 47 positioned distally of the clamping surface 44, and spaced pillars 54. The flat 47 is positioned to engage the flat 33 of the first jaw 12 when the first and second jaws 12, 14 are in their clamped position (FIG. 3). The spaced pillars 54 are positioned on opposite sides of the second locking element 46 and are positioned to direct the head 32 of the first jaw 12 towards the second locking element 46 of the second jaw 14. A distal wall of the locking element 46 defines a cam surface 60 positioned between the pillars 54. The cam surface 60 is curved outwardly in a distal direction from the clamping surface 44 downwardly towards an outer surface 62 of the second jaw 14. The cam surface 60 is configured to engage the locking element 36 of the first jaw 12 as the first jaw 12 is moved between the pillars 54 of the second jaw 14 to deform the locking element 36 in the distal direction. When the locking element 36 passes off of the cam surface 60, the locking element 36, which is formed of a resilient material, snaps inwardly such that the teeth 50 of the first locking element 36 move into engagement with the teeth 52 of the second locking element 46.

Each of the first and second jaws 14, 16 includes a boss 70 that is positioned on each side of the jaw 12, 14. The bosses 70 are positioned on a distal end 18, 42 of the first and second jaws 12, 14, respectively, and project outwardly beyond outer surfaces of the jaws 12, 14. The bosses 70 are positioned and configured to engage the jaws of an applicator (not shown) to apply the surgical clip 10 to tissue.

In embodiments, the clamping surfaces 20, 44 of the first and second jaws 12, 14, respectively, are configured to provide improved ligation. In some embodiments, the clamping surfaces 20, 44 of the first and second jaws 12, 14 includes non-slip recesses or protrusions, such as teeth, ribs, ridges, channels, chevron protrusions or recesses, etc. In certain embodiments, the clamping surface 20 of the first jaw 12 includes triangular protrusions 71 that are spaced along opposite sides of the clamping surface 20 and axially offset from adjacent protrusions 71. The protrusions 71 define a V-shaped recess 72 that extends along at least a portion of a length of the clamping surface 20 of the first jaw 12.

In embodiments, the second jaw 14 includes a triangular protrusion 74 that is positioned along at least a portion of the length of the second jaw 14. The triangular protrusion 74 is positioned to be received in the V-shaped recess 72 defined by the protrusions 71 of the first jaw 12. The triangular protrusion 74 may be defined by a series of stepped surfaces 74a. Similarly, the V-shaped recesses 72 may be defined by stepped walls of the triangular protrusions 71. Alternately, it is envisioned that other non-slip surfaces may be formed on the first and or second clamping surfaces 20, 44.

Referring to FIGS. 2-4, the surgical ligation clip 10 includes a tissue stop 80. In embodiments, the tissue stop 80 includes a first stop member 82 and a second stop member 84. The first stop member 82 is positioned at a proximal end of the clamping surface 20 of the first jaw 12 distally of the area of reduced thickness 22 and the second stop member 84 is positioned at a proximal end of the clamping surface 44 of the second jaw 14 distally of the area of reduced thickness 24. The first stop member 82 extends downwardly from a proximal end of the clamping surface 20 of the first jaw 12 towards the second jaw 14. Similarly, the stop member 84 extends upwardly from a proximal end of the clamping surface 44 of the second jaw 14 towards the first jaw 12. In embodiments, the first stop member 82 is positioned to slidably engage the second stop member 84 as the first and second jaws 12, 14 are pivoted between the unclamped position (FIG. 1) and clamped position (FIG. 3). The first and second stop members 82, 84 are dimensioned to prevent passage of tissue between the first and second jaws 12, 14 proximally beyond the clamping surfaces 20, 44 of the first and second jaws 12, 14 when the first and second jaws 12, 14 are in the unclamped position (FIG. 2) and in the clamped position (FIG. 3). Although the first stop member 82 of the tissue stop 80 is shown to be positioned distally of the second stop member 84, it is also envisioned that the position of the stop members 82, 84 could be reversed.

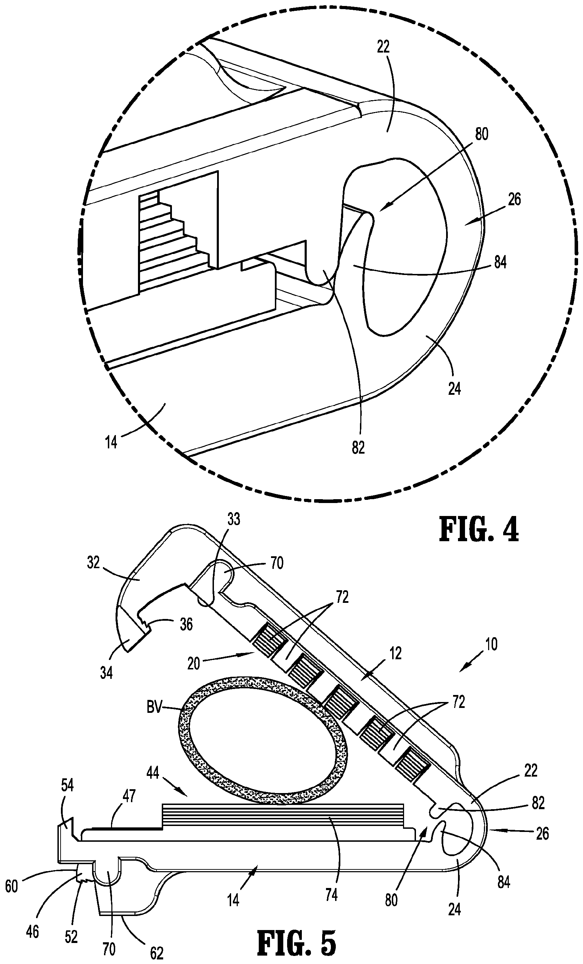

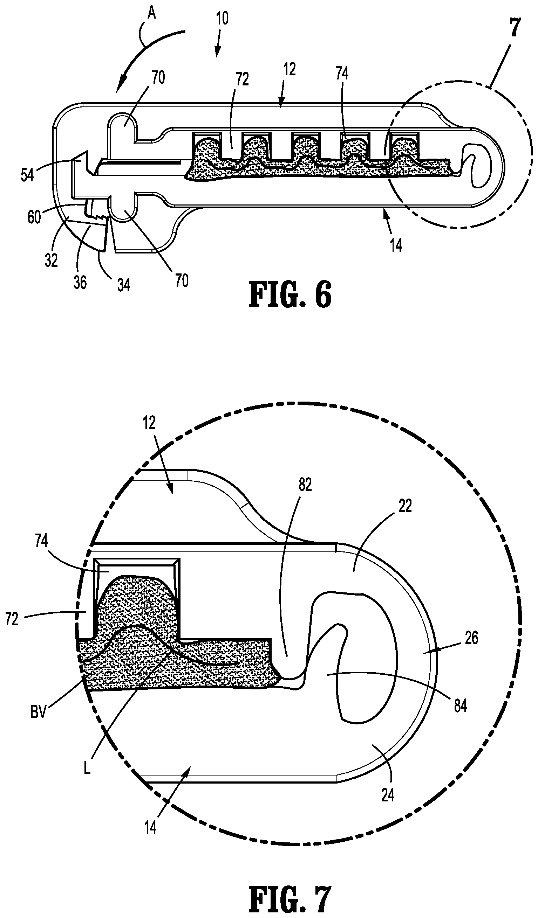

Referring to FIGS. 5 and 6, when the surgical ligation clip 10 is used to clamp a body vessel "BV", the body vessel "BV" is positioned between the clamping surfaces 20 and 44 of the first and second jaws 12, 14, and the first jaw 12 is moved in relation to the second jaw 14 in the direction indicated by arrow "A" in FIG. 6 to move the first and second jaws 12, 14 from the unclamped position (FIG. 5) to the clamped position (FIG. 6). When the first jaw 12 is pivoted towards the second jaw 14, the head 32 of the first jaw member 12 is advanced between the spaced pillars 54 of the second jaw 14 such the leading edge 34 of the first jaw 12 engages and moves along the cam surface 60 of the second jaw member 14. As the head 32 of the first jaw member 12 moves between the pillars 54 along the cam surface 60, the head 32 biased outwardly in the distal direction until the head 32 passes off of the cam surface 60. When the head 32 passes off of the cam surface 60 of the second jaw member 14, the first locking element 36 of the first jaw 12 snaps into engagement with the second locking element 46 on the second jaw 14 to secure the surgical ligation clip 10 in the clamped position about the body vessel.

As shown in FIG. 7, the tissue stop 80 is positioned to engage the body vessel "BV" as the body vessel "BV" is compressed between the first and second jaws 12, 14 to prevent the body vessel "BV" from moving proximally off of the proximal end of the clamping surfaces 20, 44 of the first and second jaws 12, 14, respectively. Retaining the body vessel "BV" at a location between the clamping surfaces 20, 44 of the first and second jaws 12, 14 reduces any likelihood that a lumen "L" defined by the body vessel "BV" will not be sealed when the jaws 12, 14 of the surgical ligation clip 10 are in the clamped position.

In embodiments, the surgical ligation clip 10 may be comprised of a bioabsorbable polymeric material. Examples of suitable bioabsorbable polymers include acetal polyoxymethylene (POM), polyethylene terephthalate (PET), polybutylene terephthalate (PBT), polyoxymethylene, polyetheretherketone (PEEK), polypropylene, and polyethylene or other thermoplastic materials having similar properties that can be injection-molded. The clip may also be comprised of polymer material in combination with radiolucent metal alloys. Alternately, other materials may be used to form the clip 10 including biocompatible metals, plastics and composites. In embodiments, the material selected is resilient.

Persons skilled in the art will understand that the devices and methods specifically described herein and illustrated in the accompanying drawings are non-limiting exemplary embodiments. It is envisioned that the elements and features illustrated or described in connection with one exemplary embodiment may be combined with the elements and features of another without departing from the scope of the present disclosure. As well, one skilled in the art will appreciate further features and advantages of the disclosure based on the above-described embodiments. Accordingly, the disclosure is not to be limited by what has been particularly shown and described, except as indicated by the appended claims.

The invention may be described by reference to the following numbered paragraphs:-
1. A surgical ligation clip comprising:
   a first jaw having a distal end, a proximal end, and a first clamping surface;
   a second jaw having a distal end, a proximal end, and a second clamping surface, the second clamping surface being in opposition to the first clamping surface;
   a living hinge pivotably coupling the proximal end of the first jaw to the proximal end of the second jaw, wherein the first and second jaws are movable in relation to each other between an unclamped position and a clamped position; and
   a tissue stop supported on the surgical ligation clip, the tissue stop being positioned to prevent tissue from being positioned between the first and second jaws proximally of the first and second clamping surfaces when the first and second jaws are in the unclamped position.
2. The surgical ligation clip of paragraph 1, wherein the tissue stop includes a first stop member supported on the first jaw, the first stop member extending from a proximal end of the first clamping surface towards the second jaw.
3. The surgical ligation clip of paragraph 2, wherein the tissue stop includes a second stop member, the second stop member extending from a proximal end of the second clamping surface of the second jaw towards the first jaw.
4. The surgical ligation clip of paragraph 3, wherein the first stop member is positioned to slidably engage the second stop member when the first and second jaws are moved in relation to each other from the unclamped position to the clamped position.
5. The surgical ligation clip of paragraph 1, wherein the first jaw includes a first locking element and the second jaw member includes a second locking element, the first locking element being movable into engagement with the second locking element to retain the first and second jaws in the clamped position.
6. The surgical ligation clip of paragraph 5, wherein the distal end of the first jaw includes a head, the head supporting the first locking element.
7. The surgical ligation clip of paragraph 5, wherein the head includes a pointed leading end that is configured to penetrate tissue.
8. The surgical ligation clip of paragraph 1, wherein each of the first and second jaws includes a pair of bosses, each of the pair of bosses being configured to engage an applicator to apply the surgical ligation clip to tissue.
9. The surgical ligation clip of paragraph 1, wherein each of the pair of bosses projects outwardly beyond an outer surface of one of the first and second jaws.
10. The surgical ligation clip of paragraph 1, wherein the clamping surfaces of the first and second jaws include a non-slip structure.
11. The surgical ligation clip of paragraph 1, wherein the non-slip structure of the first clamping surface includes triangular protrusions, the triangular protrusions are spaced along opposite sides of the clamping surface and are axially offset from adjacent protrusions.
12. The surgical ligation clip of paragraph 11, wherein the triangular protrusions define a centrally located V-shaped recess that extends along at least a portion of the first clamping surface of the first jaw.
13. The surgical ligation clip of paragraph 12, wherein the second jaw includes a centrally located triangular protrusion that is positioned along at least a portion of the length of the second jaw, the centrally located triangular protrusion being positioned to be received in the centrally located V-shaped recess defined by the protrusions of the first jaw.
14. The surgical ligation clip of paragraph 1, wherein the surgical ligation clip is formed from a biocompatible polymeric material.
15. The surgical ligation clip of paragraph 14, wherein the biocompatible polymeric material is selected from the group consisting of acetal polyoxymethylene (POM), polyethylene terephthalate (PET), polybutylene terephthalate (PBT), polyoxymethylene, polyetheretherketone (PEEK), polypropylene, and polyethylene.
16. The surgical ligation clip of paragraph 1, wherein the surgical ligation clip is formed from a biocompatible material selected from the group consisting of metals, plastics and composites.

## Claims

1. A surgical ligation clip comprising:
a first jaw having a distal end, a proximal end, and a first clamping surface;
a second jaw having a distal end, a proximal end, and a second clamping surface, the second clamping surface being in opposition to the first clamping surface;
a living hinge pivotably coupling the proximal end of the first jaw to the proximal end of the second jaw, wherein the first and second jaws are movable in relation to each other between an unclamped position and a clamped position; and
a tissue stop supported on the surgical ligation clip, the tissue stop being positioned to prevent tissue from being positioned between the first and second jaws proximally of the first and second clamping surfaces when the first and second jaws are in the unclamped position.

2. The surgical ligation clip of claim 1, wherein the tissue stop includes a first stop member supported on the first jaw, the first stop member extending from a proximal end of the first clamping surface towards the second jaw.

3. The surgical ligation clip of claim 2, wherein the tissue stop includes a second stop member, the second stop member extending from a proximal end of the second clamping surface of the second jaw towards the first jaw.

4. The surgical ligation clip of claim 3, wherein the first stop member is positioned to slidably engage the second stop member when the first and second jaws are moved in relation to each other from the unclamped position to the clamped position.

5. The surgical ligation clip of any preceding claim, wherein the first jaw includes a first locking element and the second jaw member includes a second locking element, the first locking element being movable into engagement with the second locking element to retain the first and second jaws in the clamped position.

6. The surgical ligation clip of claim 5, wherein the distal end of the first jaw includes a head, the head supporting the first locking element.

7. The surgical ligation clip of claim 5 or claim 6, wherein the head includes a pointed leading end that is configured to penetrate tissue.

8. The surgical ligation clip of any preceding claim, wherein each of the first and second jaws includes a pair of bosses, each of the pair of bosses being configured to engage an applicator to apply the surgical ligation clip to tissue.

9. The surgical ligation clip of any preceding claim, wherein each of the pair of bosses projects outwardly beyond an outer surface of one of the first and second jaws.

10. The surgical ligation clip of any preceding claim, wherein the clamping surfaces of the first and second jaws include a non-slip structure.

11. The surgical ligation clip of claim 1, wherein the non-slip structure of the first clamping surface includes triangular protrusions, the triangular protrusions are spaced along opposite sides of the clamping surface and are axially offset from adjacent protrusions.

12. The surgical ligation clip of claim 11, wherein the triangular protrusions define a centrally located V-shaped recess that extends along at least a portion of the first clamping surface of the first jaw.

13. The surgical ligation clip of claim 12, wherein the second jaw includes a centrally located triangular protrusion that is positioned along at least a portion of the length of the second jaw, the centrally located triangular protrusion being positioned to be received in the centrally located V-shaped recess defined by the protrusions of the first jaw.

14. The surgical ligation clip of any preceding claim, wherein the surgical ligation clip is formed from a biocompatible polymeric material; preferably, wherein the biocompatible polymeric material is selected from the group consisting of acetal polyoxymethylene (POM), polyethylene terephthalate (PET), polybutylene terephthalate (PBT), polyoxymethylene, polyetheretherketone (PEEK), polypropylene, and polyethylene.

15. The surgical ligation clip of any preceding claim, wherein the surgical ligation clip is formed from a biocompatible material selected from the group consisting of metals, plastics and composites.
